# FASCICULE DE BREVET EUROPEEN

(11) **EP 0 847 786 B1**
(45) Date de publication et mention de la délivrance du brevet: **18.07.2001**
(21) Numéro de dépôt: 97402981.1
(22) Date de dépôt: 10.12.1997
(51) Int. Cl.: B01D 39/18

(54) **Papier chargé de filtration de gaz**
Füllstoffhaltiges Papier zum Filtrieren von Gas
filled paper for gas filtration

(30) Priorité: 12.12.1996 FR 9615295
(43) Date de publication de la demande: 17.06.1998
(73) Titulaire: ELF ANTAR FRANCE, 92400 Courbevoie (FR); Ahlstrom Industries SA, 94400 Vitry sur Seine Cedex (FR)
(72) Inventeur: Navarre, François-Pierre, 69260 Charbonnière Les Bains (FR); Bossand, Bernard, 69360 Communay (FR); Girard, Pierre, 38330 Saint Ismier (FR); Dussaud, Joseph, 38780 Pont-Evêque (FR)
(74) Mandataire: Jolly, Jean-Pierre

(56) Documents cités:
- EP-A- 0 207 588
- DE-U- 7 720 093
- US-A- 4 988 440
- US-A- 5 202 296
- US-A- 5 352 274
- DATABASE WPI Section Ch, Week 9509 Derwent Publications Ltd., London, GB; Class D22, AN 95-063412 XP002038798 & JP 06 339 629 A (KONDO KOGYO KK) , 13 décembre 1994

## Description

### DOMAINE TECHNIQUE

La présente invention concerne un papier de filtration de gaz ayant une perméance à l'air élevée et de fortes capacités d'adsorption et d'absorption.

Elle trouve son application pour la filtration et la purification de gaz en général et d'air en particulier, notamment dans le traitement des rejets de nombreuses industries, dans l'automobile, l'habitat, l'hôtellerie, la restauration, les immeubles de bureau, les hôpitaux, les lieux publics. Le papier de l'invention permet d'éliminer à faible coût les particules solides, les vapeurs de liquides indésirables, les micro-organismes et les agents pathogènes en suspension dans les gaz traités.

### ETAT DE LA TECHNIQUE ANTERIEURE

Des papiers filtres sont couramment utilisés comme média filtrant pour la filtration et la purification des gaz. Les papiers connus ont l'inconvénient de présenter une perméance à l'air faible, qui se traduit pour un débit donné de gaz à traiter au moyen d'une surface de papier donnée, par une perte de charge élevée, qu'il faut compenser par une surpression du gaz pour assurer sa circulation. Pour des volumes de gaz importants, il en résulte un coût d'exploitation élevé.

Une autre solution consiste à augmenter la surface de papier ce qui conduit à un dimensionnement supérieur des équipements, donc à des investissements élevés et des encombrements importants.

Selon les techniques connues on est amené à utiliser des papiers à faible grammage de 15 à 50 g/m2, et à superposer plusieurs feuilles pour compenser les effets des dimensions importantes des espaces libres entre les fibres pouvant atteindre 200 microns. Cette technique est utilisée notamment pour la fabrication de filtres d'aspirateurs de poussières. Ces papiers de faibles grammages doivent être traités chimiquement pour qu'ils présentent une résistance mécanique suffisante pour ce type d'application. Le coût de fabrication de ces papiers est élevé.

De plus tous les papiers filtrants connus, du fait qu'ils sont utilisés en feuilles de faibles épaisseurs ont des capacités d'adsorption et d'absorption très faibles et généralement insuffisantes pour que des réactions chimiques ou catalytiques indispensables à la purification de certaines suspensions puissent se produire pendant le temps de passage des gaz au travers des feuilles.

Une autre technique connue de filtration consiste à faire passer les gaz à traiter au travers de poudres telles que des charbons actifs disposés en lits fixes. Ces poudres se chargent en polluants et au fur et à mesure qu'elles se chargent elles perdent de leur efficacité. Quand elles sont saturées elles doivent être régénérées. Les installations qui permettent d'assurer les opérations du cycle d'adsorption désorption sont coûteuses. De plus, à chaque cycle, le rendement et la sélectivité des poudres diminuent.

### DESCRIPTION DE L'INVENTION

L'invention a justement pour but de remédier à ces inconvénients. A cette fin elle propose un papier de filtration de gaz présentant une perméance à l'air élevée, à forte capacité d'adsorption et d'absorption, à base de fibres cellulosiques caractérisé en ce qu'il comporte en outre au moins une matière organique pulvérulente dont 95% des particules ont une granulométrie comprise entre 1 et 150 microns, dans une proportion comprise entre 5 et 75% du poids du papier incluant ladite matière organique.

Selon une autre caractéristique de l'invention la matière organique comprend un bois torréfié micronisé de granulométrie comprise entre 50 et 150 microns.

Selon une autre caractéristique de l'invention la matière organique comprend une poudre de résineux.

Selon une autre caractéristique le papier de l'invention comporte en outre au moins un matériau inorganique dans une proportion de 0,2 à 70% du poids du papier incluant ladite matière organique et ledit matériau inorganique.

Selon une autre caractéristique de l'invention le matériau inorganique est un produit dérivé de la silice, sous forme de poudre.

Selon une autre caractéristique de l'invention le matériau inorganique est un produit dérivé de l'alumine, sous forme de poudre.

Selon une autre caractéristique de l'invention le matériau inorganique est un catalyseur pulvérulent de granulométrie comprise entre 2 et 100 microns.

Selon une autre caractéristique, le papier de l'invention comporte en outre un agent bactéricide.

Selon une autre caractéristique, le papier de l'invention comporte en outre un agent virocide.

Selon une autre caractéristique, le papier de l'invention a subi un traitement pour le rendre oléophile.

Selon une autre caractéristique, le papier de l'invention a subi un traitement pour le rendre hydrophobe.

Selon une autre caractéristique de l'invention, le papier chargé est utilisé pour filtrer et purifier des gaz.

Selon une autre caractéristique de l'invention, le papier chargé est utilisé pour filtrer et purifier un gaz d'échappement de moteur à combustion interne.

Selon une autre caractéristique de l'invention, le papier chargé est utilisé pour filtrer et purifier de l'air de ventilation d'immeuble.

Selon une autre caractéristique de l'invention, le papier chargé est utilisé pour filtrer et purifier de l'air vicié de cuisine

Selon une autre caractéristique de l'invention, le papier chargé est utilisé pour filtrer et purifier de l'air de ventilation d'un véhicule routier.

D'autres caractéristiques et avantages de l'invention apparaîtront mieux à la lecture de l'exposé détaillé et des exemples qui vont suivre.

### EXPOSE DETAILLE DE L'INVENTION

L'invention a pour objet un papier chargé en poudres, destiné à filtrer et à purifier des gaz. Le papier de l'invention est réalisé à partir de fibres cellulosiques auxquelles sont ajoutées des poudres. Le média ainsi obtenu présente une perméance à l'air élevée et des propriétés d'absorption et d'adsorption liées à la nature des poudres.

Ce résultat est illustré par les tableaux suivants :

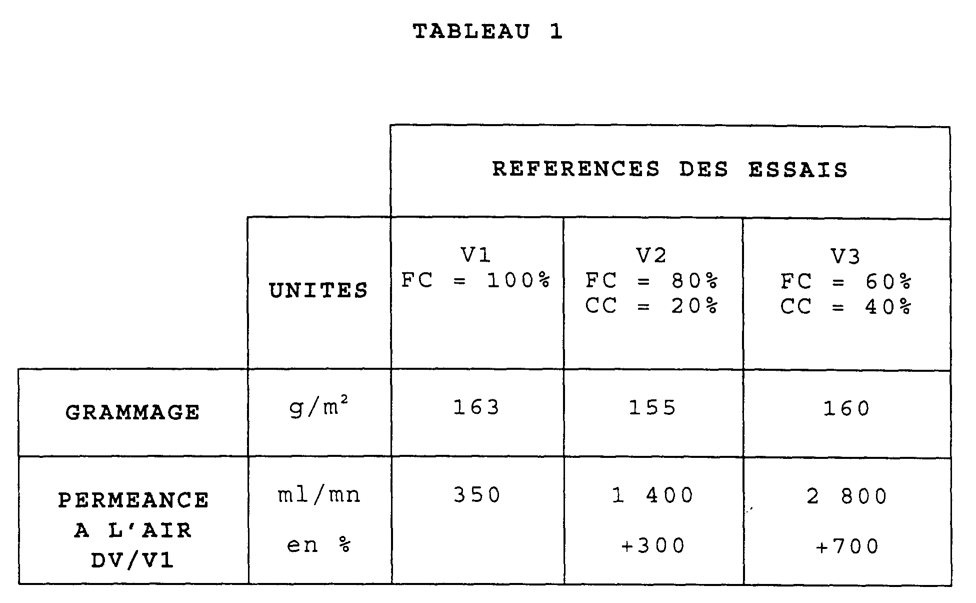

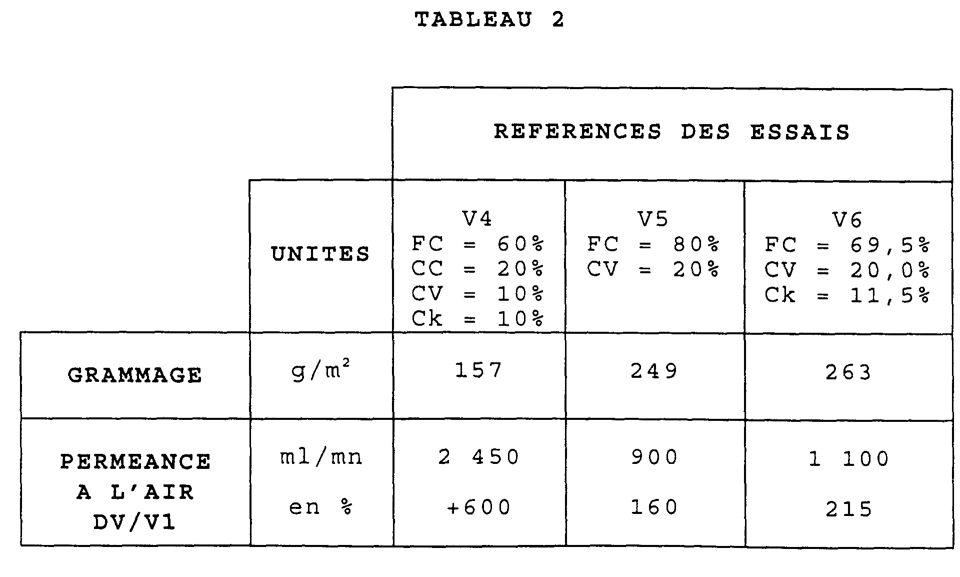

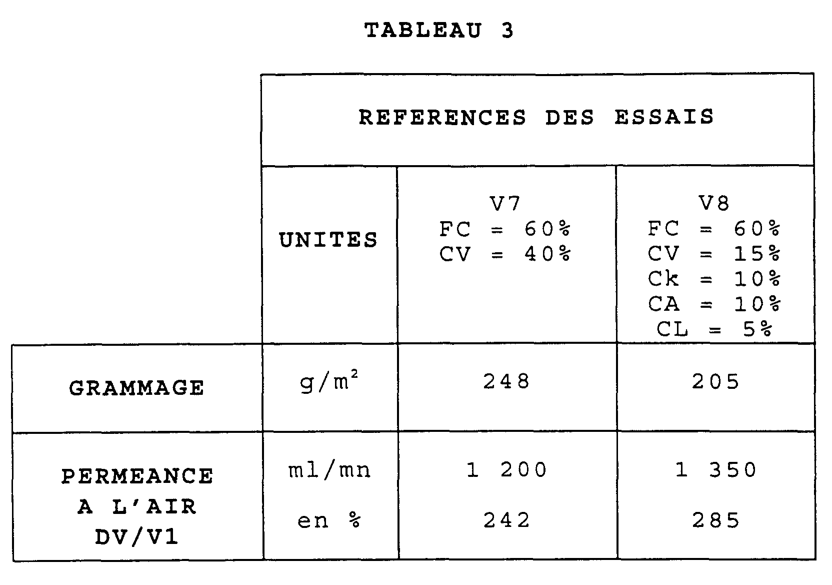

Dans lesquels :
- V1, V2, V3, V4,V5, V6, V7 et V8.sont des variantes de composition du papier
- FC signifie fibres cellulosiques.
- CC signifie catalyseur usé de craqueur catalytique.
- CV signifie charge végétale (poudre d'épicéa de granulométrie comprise entre 100 et 150 microns).
- Ck signifie coke de charbon de granulométrie inférieure à 160 microns.
- CL signifie clarcel
- CA signifie charbon actif
- DV / V1 représente l'augmentation de perméance à l'air pour chaque essais par rapport à l'essai V1.

La perméance à l'air est mesurée au moyen d'un appareil Bendtsen conformément à la norme NF Q 03.076, elle représente l'aptitude du papier à être traversé par l'air.

Le grammage étant sensiblement le même on observe une augmentation relative de la perméance à l'air de 700 %, ce qui est considérable.

Ces résultats montrent que le papier de l'invention est un matériau filtrant dont on peut adapter les caractéristiques au gaz à filtrer, en agissant sur sa composition en qualité et en quantité.

Le papier de l'invention est utilisable comme média filtrant avec des dispositifs connus mettant en oeuvre des procédés connus. A titre d'exemple on peut citer les filtres à bande à déroulement de papier.

Des papiers filtrants ayant des propriétés similaires sont obtenus en les chargeant avec des poudres de bois torréfiés et micronisés.

Selon une autre caractéristique de l'invention, le papier de filtration est chargé d'agents qui modifient son pH, par exemple du sulfate d'alumine, de l'acide sulfurique, des résines échangeuses d'ions.

Pour traiter des gaz qui nécessitent des capacités d'échange d'ions ou des capacités élevées d'adsorption et d'absorption selon une autre caractéristique de l'invention on ajoute aux fibres cellulosiques des matières pulvérulentes minérales dérivées de la silice, telles que des silicates, de la silice naturelle ou des silicoaluminates cristallisés.

Selon une autre caractéristique de l'invention le papier est chargé de catalyseur usé de craqueur catalytique, qui présentent une surface spécifique de 10 m2/g et une porosité de 0,5 cm3/g et dont les caractéristiques granulométriques sont les suivantes :

| **TAILLE DES PARTICULES DE CATALYSEUR EN MICRONS** | **PROPORTIONS EN % VOLUME** |
|---|---|
| 0 - 40 | 10 |
| 40 - 80 | 55 |
| 80 - 105 | 20 |
| 105 - 150 | 10 |
| > 150 | 5 |

Une particularité intéressante de ce type de catalyseur est qu'il présente des pores de rayon moyen de l'ordre de 20 nanomètres qui sont de véritables pièges à molécules.

Le papier de filtration chargé en catalyseur de craqueur catalytique a aussi l'avantage de bien résister à des températures de mise en oeuvre de l'ordre de 100°C, d'être doté de propriétés oléophiles qui en font un bon coalesceur d'huiles et d'avoir une bonne résistance mécanique.

Une autre caractéristique de l'invention est que le papier chargé peut être traité de manière connue pour le rendre oléophobe, oléophile, ignifuge afin de l'adapter au gaz à filtrer.

En fonction des besoins le papier chargé en poudres peut aussi comporter des agents bactéricides et/ou virocides. Ces agents peuvent être introduits lors de la fabrication du papier ou au moment de son utilisation par adsorption de produits bactéricides comme le formol et l'isotiasolidine ou par greffage chimique. Ce type de papier trouve un intérêt particulier pour la filtration d'air de climatisation dans les ateliers, les salles blanches, les immeubles de bureaux et les hôpitaux. Les papiers chargés après utilisation sont détruits par incinération ce qui évite tout risque de contamination.

Un des principaux avantages de l'invention est de pouvoir utiliser un papier de filtration dont les caractéristiques sont appropriées à chaque application, en choisissant la fibre cellulosique naturelle ou synthétique et la ou les poudres les mieux adaptées.

On pourra mieux apprécier cet avantage de l'invention à la lecture des exemples suivants.

### EXEMPLE 1 : Filtration d'air de climatisation

Un local ventilé ou climatisé occupé par du personnel nécessite une circulation d'air frais à un débit de l'ordre de 5 l/s par occupant et de 7 l/s par occupant fumeur ou aillant une activité physique moyenne. Généralement l'air renouvelé véhicule des poussières dont les dimensions sont comprises entre 1 et quelques dizaines de nanomètres. Ces poussières s'accumulent dans les conduites sur les supports et grilles des aérateurs et constituent des niches favorables au développement de germes pathogènes dangereux pour la santé humaine.

Le papier chargé filtrant le mieux adapté à cette application à la composition suivante :
- 70% de fibres obtenues à partir de résineux écrus ou blanchis
- 30%de poudres organiques végétales de granulométrie comprise entre 100 et 150 microns

La perméance de ce papier chargé est de 1 000 ml/mn soit environ 200 fois plus élevée que celle d'un papier classique.

Il peut être utilisé comme média filtrant avec des appareils conventionnels.

### EXEMPLE 2 : Filtration et purification d'air de cuisine avant rejet à l'extérieur ou avant recyclage.

Dans cette application l'objectif visé est de fixer les vapeurs grasses et de désodoriser l'air.

Le papier chargé filtrant le mieux adapté à cette application à la composition suivante :
- 50% de fibres obtenues à partir de fibres cellulosiques blanchies,
- 25% de poudres minérales de silice alumine de granulométrie comprise entre 5 et 100 microns.
- 25%de poudres organiques végétales de granulométrie comprise entre 20 et 150 microns.

La perméance de ce papier chargé est de 1 100 ml/mn soit environ 3,1 fois plus élevée que celle d'un papier classique.

Il peut être utilisé comme média filtrant avec des hottes conventionnelles.

### EXEMPLE 3 : Filtration de l'air d'appoint pour la ventilation des véhicules.

L'objectif est de retenir les poussières et de fixer les hydrocarbures imbrûlés contenus dans l'air pollué des villes.

Le papier chargé filtrant le mieux adapté à cette application à la composition suivante :
- 60% de fibres obtenues à partir de fibres écrues
- 20% de poudres végétales issues de résineux, de granulométrie comprise entre 50 et 150 microns.
- 20% de poudres minérales, de granulométrie comprise entre 5 et 150 microns.

La perméance de ce papier chargé est de 2 500 ml/mn soit environ 7 fois plus élevée que celle d'un papier classique.

Il peut être utilisé comme média filtrant avec des cartouches conventionnelles qui sont changées en même temps que les autres filtres du véhicule.

### EXEMPLE 4 : Nettoyage des surfaces de stations services pour améliorer l'environnement.

Pour cette application le papier chargé de l'invention est utilisé comme absorbeur d'hydrocarbures autour des zones de distribution de gazole, essence et autres carburants.

Le papier doit être ignifugé et antistatique pour cette utilisation.

Le papier chargé filtrant le mieux adapté à cette application à la composition suivante :
- 40% de fibres écrues
- 10% de fibres recyclées
- 20% de produits ignifugeants constitués par un tiers de phosphate d'ammonium, un tiers de sulfonate d'ammonium et un tiers de sulfate d'ammonium
- 30% de poudres végétales de granulométrie comprise entre 100 et 150 microns.

La perméance de ce papier chargé est de 700 ml/mn soit environ 2 fois plus élevée que celle d'un papier classique.

## Revendications

1. Papier chargé de filtration de gaz présentant une perméance à l'air élevée, à forte capacité d'adsorption et d'absorption, à base de fibres cellulosiques comportant en outre au moins une matière organique pulvérulente dont 95% des particules ont une granulométrie comprise entre 1 et 150 microns, dans une proportion comprise entre 5 et 75% du poids du papier incluant ladite matière organique caractérisé en ce que ladite matière organique comprend au moins un élément choisi dans le groupe constitué par un bois torréfié micronisé de granulométrie comprise entre 50 et 150 microns et une poudre de résineux.

2. Papier selon la revendication 1, caractérisé en ce qu'il comporte en outre au moins un matériau inorganique dans une proportion de 0,2 à 70% du poids du papier incluant ladite matière organique et ledit matériau inorganique.

3. Papier selon la revendication 2, caractérisé en ce que le matériau inorganique est un produit dérivé de la silice, sous forme de poudre.

4. Papier selon la revendication 2, caractérisé en ce que le matériau inorganique est un produit dérivé de l'alumine, sous forme de poudre.

5. Papier selon la revendication 2, caractérisé en ce que le matériau inorganique est un catalyseur pulvérulent de granulométrie comprise entre 2 et 100 microns.

6. Papier selon l'une des revendications 1 à 5, caractérisé en ce qu'il comporte en outre un agent bactéricide.

7. Papier selon l'une des revendications 1 à 6, caractérisé en ce qu'il comporte en outre un agent virocide.

8. Papier selon l'une des revendications 1 à 7, caractérisé en ce que le papier a subi un traitement pour le rendre oléophile.

9. Papier selon l'une des revendications 1 à 7, caractérisé en ce que le papier a subi un traitement pour le rendre hydrophobe.

10. Utilisation du papier selon l'une des revendications 1 à 9 caractérisée en ce qu'elle consiste à filtrer et purifier des gaz.

11. Utilisation selon la revendication 10 caractérisée en ce que le gaz à filtrer et purifier est un gaz d'échappement de moteur à combustion interne.

12. Utilisation selon la revendication 10 caractérisée en ce que le gaz à filtrer et purifier est de l'air de ventilation d'immeuble.

13. Utilisation selon la revendication 10 caractérisée en ce que le gaz à filtrer et purifier est de l'air vicié de cuisine

14. Utilisation selon la revendication 10 caractérisée en ce que le gaz à filtrer et purifier est de l'air de ventilation d'un véhicule routier.

## Claims

1. A loaded paper for filtering gas having high air permeability and a high adsorption and absorption capacity, based on cellulose fibres, additionally comprising at least one powdered organic material of which 95 % of the particles have a particle size of between 1 and 150 microns, in a proportion of between 5 and 75 % of the weight of the paper including said organic material, characterised in that said organic material comprises at least one element selected from the group comprising a micronised roasted wood of a particle size of between 50 and 150 microns and a resinous powder.

2. A paper according to claim 1, characterised in that it further comprises at least one inorganic material in a proportion of 0.2 to 70 % by weight of the paper including said organic material and said inorganic material.

3. A paper according to claim 2, characterised in that the inorganic material is a product derived from silica, in the form of a powder.

4. A paper according to claim 2, characterised in that the inorganic material is a product derived from alumina, in the form of a powder.

5. A paper according to claim 2, characterised in that the inorganic material is a powdered catalyst of a particle size of between 2 and 100 microns.

6. A paper according to any one of claims 1 to 5, characterised in that it further comprises a bactericidal agent.

7. A paper according to any one of claims 1 to 6, characterised in that it further comprises a virocidal agent.

8. A paper according to any one of claims 1 to 7, characterised in that the paper has undergone a treatment to render it oleophilic.

9. A paper according to any one of claims 1 to 7, characterised in that the paper has undergone a treatment to render it hydrophobic.

10. Use of the paper according to any one of claims 1 to 9, characterised in that it comprises filtering and purifying gases.

11. Use according to claim 10, characterised in that the gas to be filtered and purified is an exhaust gas from an internal-combustion engine.

12. Use according to claim 10, characterised in that the gas to be filtered and purified is the ventilation air of a building.

13. Use according to claim 10, characterised in that the gas to be filtered and purified is the contaminated air from cooking.

14. Use according to claim 10, characterised in that the gas to be filtered and purified is the ventilation air of a road vehicle.

## Patentansprüche

1. Füllstoffhaltiges Papier zum Filtrieren von Gas, mit einer erhöhten Luftdurchlässigkeit, einem hohen Adsorptions- und Absorptionsvermögen, auf der Basis von Cellulosefasern, das außerdem mindestens eine pulverförmige organische Substanz, von der 95 % der Teilchen eine Korngrößenverteilung zwischen 1 und 150 *µ*m haben, in einer Menge zwischen 5 und 75 % des Gewichts des die organische Substanz enthaltenden Papiers enthält, dadurch gekennzeichnet, dass die organische Substanz mindestens ein Element enthält, das aus der Gruppe, bestehend aus einem gedörrten und mikronisierten Holz, mit einer Korngrößenverteilung zwischen 50 und 150 *µ*m , und einem harzhaltigen Pulver ausgewählt ist.

2. Papier nach Anspruch 1, dadurch gekennzeichnet, dass es außerdem mindestens ein anorganisches Material in einer Menge von 0.2 bis 70 % des Gewichts des die organische Substanz und das anorganische Material enthaltenden Papiers enthält.

3. Papier nach Anspruch 2, dadurch gekennzeichnet, dass das anorganische Material ein von Kieselsäure abgeleitetes Produkt in Pulverform darstellt.

4. Papier nach Anspruch 2, dadurch gekennzeichnet, dass das anorganische Material ein von Aluminiumoxid abgeleitetes Produkt in Pulverform darstellt.

5. Papier nach Anspruch 2, dadurch gekennzeichnet, dass das anorganische Material einen pulverförmigen Katalysator mit einer Korngrößenverteilung zwischen 2 und 100 *µ*m darstellt.

6. Papier nach einem der Ansprüche 1 bis 5, dadruch gekennzeichnet, dass es außerdem ein bakterizides Mittel enthält.

7. Papier nach einem der Ansprüche 1 bis 6, dadurch gekennzeichnet, dass es außerdem ein virizides Mittel enthält.

8. Papier nach einem der Ansprüche 1 bis 7, dadurch gekennzeichnet, dass das Papier einer Oleophilisierungsbehandlung unterzogen worden ist.

9. Papier nach einem der Ansprüche 1 bis 7, dadurch gekennzeichnet, dass das Papier einer Hydophobierungsbehandlung unterzogen worden ist.

10. Verwendung des Papiers nach einem der Ansprüche 1 bis 9 zur Filtration und Gasreinigung.

11. Verwendung nach Anspruch 10, dadurch gekennzeichnet, dass das zu filtrierende und zu reinigende Gas ein Abgas von Verbrennungsmotoren darstellt.

12. Verwendung nach Anspruch 10, dadurch gekennzeichnet, dass das zu filtrierende und zu reinigende Gas Luft zur Belüftung von Gebäuden darstellt.

13. Verwendung nach Anspruch 10, dadurch gekennzeichnet, dass das zu filtrierende und zu reinigende Gas verbrauchte Küchenluft darstellt.

14. Verwendung nach Anspruch 10, dadurch gekennzeichnet, dass das zu filtrierende und zu reinigende Gas die Ventilationsluft eines Straßenfahrzeugs darstellt.
